# EUROPÄISCHE PATENTSCHRIFT

(11) **EP 2 104 454 B1**
(45) Veröffentlichungstag und Bekanntmachung des Hinweises auf die Patenterteilung: **16.03.2011**
(21) Anmeldenummer: 08804112.4
(22) Anmeldetag: 12.09.2008
(51) Int. Cl.: A61B 6/00

(54) **VERFAHREN ZUM POSITIONIEREN DER BRUST FÜR EINE BIOPSIE IN EINER MAMMOGRAPHIEEINRICHTUNG UND MAMMOGRAPHIEEINRICHTUNG ZUM DURCHFÜHREN DES VERFAHRENS**
METHOD FOR POSITIONING THE BREAST FOR A BIOPSY IN A MAMMOGRAPHY DEVICE AND MAMMOGRAPHY DEVICE FOR CARRYING OUT THE METHOD
PROCEDE POUR POSITIONNER UN SEIN DANS UNE MAMMOGRAPHIE EN VUE D'UNE BIOPSIE ET MAMMOGRAPHIE DESTINE A REALISER LE PROCEDE

(30) Priorität: 20.12.2007 DE 102007061592
(43) Veröffentlichungstag der Anmeldung: 30.09.2009
(73) Patentinhaber: Siemens Aktiengesellschaft, 80333 München (DE)
(72) Erfinder: FISCHER, Daniel, 91052 Erlangen (DE); HOFMANN, Carina, 91058 Erlangen (DE); MERTELMEIER, Thomas, 91058 Erlangen (DE)
(86) Internationale Anmeldenummer: PCT/EP2008/062148
(87) Internationale Veröffentlichungsnummer: WO 2009/080378

(56) Entgegenhaltungen:
- EP-A- 1 419 735
- WO-A-2005/110230
- WO-A-2005/110233
- WO-A-2007/023050
- US-A1- 2006 093 084
- US-A1- 2007 139 799

## Beschreibung

Die Erfindung bezieht sich auf ein Verfahren zum Positionieren der Brust für eine Biopsie in einer Mammographieeinrichtung. Außerdem bezieht sich die Erfindung auf eine Mammographieeinrichtung zum Durchführen dieses Verfahrens.

Wenn im Rahmen einer Mammographieuntersuchung im Röntgenbild der Brust eine krankhafte Gewebsveränderung oder Läsion festgestellt wird, ist es zur eindeutigen Diagnosestellung in vielen Fällen erforderlich, eine Gewebeprobe zu entnehmen und diese histologisch zu untersuchen. Bei der stereotaktischen Biopsie wird hierzu die Brust in einer Mammographieeinrichtung zwischen einer Kompressionsplatte und einer Lagerplatte komprimiert und fixiert. Die Kompressionsplatte enthält eine rechteckige Aussparung, durch die das Biopsieinstrument in die Brust eingeführt werden kann. Um sicherzustellen, dass die Gewebeprobe an der richtigen Stelle entnommen wird, werden während des Eingriffes Röntgenaufnahmen aus unterschiedlichen Richtungen erstellt und die Läsion in den Röntgenaufnahmen markiert. Auf diese Weise lässt sich die genaue Einstichposition und Einstichtiefe des Biopsieinstrumentes bestimmen. Die Brust muss nun bereits vor diesen Röntgenaufnahmen so positioniert werden, dass sich die Projektion der Läsion innerhalb der Projektion der rechteckigen Aussparung befindet. Zur richtigen Positionierung der Brust ist es deshalb notwendig vor der Aufnahme der Röntgenbilder aus unterschiedlichen Richtungen eine oder mehrere Röntgenbilder aufzunehmen, die ausschließlich dazu dienen, die Brust richtig zwischen Kompressionsplatte und Lagerplatte zu positionieren. Jede derartige Röntgenaufnahme bedeutet jedoch eine zusätzliche Strahlenbelastung für die Patientin; siehe z. Bsp. WO-A-2007/023050.

Der Erfindung liegt deshalb die Aufgabe zu Grunde, ein Verfahren zum Positionieren der Brust für eine Biopsie in einem Mammographiegerät anzugeben, mit dem die Anzahl der zur korrekten Positionierung erforderlichen Röntgenbilder verringert ist. Außerdem liegt der Erfindung die Aufgabe zu Grunde, ein Mammographiegerät zum Durchführen des Verfahrens anzugeben.

Hinsichtlich des Verfahrens wird die Aufgabe gemäß der Erfindung gelöst mit einem Verfahren mit den Merkmalen des Patentanspruches 1. Das Verfahren zum Positionieren der Brust für eine Biopsie in einer Mammographieeinrichtung umfasst die folgenden Merkmale:
a) aus zumindest einem Röntgenbild der Brust wird eine virtuelle Maske erzeugt, die den vom Brustgewebe abgedeckten Bildbereich wiedergibt und in der ein innerhalb des Brustgewebes liegender und zur Biopsie vorgesehener Bereich markiert ist,
b) die Brust wird für die Biopsie in der Mammographieeinrichtung zwischen einer Lagerplatte und einer mit einer Aussparung zum Einführen eines Biopsieinstrumentes versehenen Kompressionsplatte positioniert,
c) die Maske und die Kompressionsplatte werden derart überlagert, dass der markierte Bereich innerhalb der Aussparung angeordnet ist,
d) die Brust wird solange verschoben bis ihre Lage und Kontur zumindest annähernd mit der Lage und Kontur der Maske übereinstimmt.

Durch diese Maßnahme wird die Anzahl der zur korrekten Positionierung der Brust für die Biopsie erforderlichen Röntgenaufnahmen deutlich verringert, wobei im günstigsten Fall die Notwendigkeit für die Aufnahme eines Röntgenbildes vollständig entfällt, da für die Erzeugung der Maske auf ein gespeichertes Röntgenbild zurückgegriffen werden kann, das im Rahmen einer Voruntersuchung gewonnen worden ist.

Wenn von der Maske im Bereich der Kompressionsplatte ein sichtbares Bild, insbesondere ein auf die Kompressionsplatte projiziertes Projektionsbild derart erzeugt wird, dass der markierte Bereich im Projektionsbild der Maske innerhalb der Aussparung angeordnet ist, und die Brust solange verschoben wird, bis ihre Lage und Kontur zumindest annähernd mit der Lage und Kontur des Bildes oder Projektionsbildes der Maske übereinstimmt, ist eine besonders einfache und intuitive Positionierung der Brust unmittelbar durch Vergleich des Bildes oder des Projektionsbildes mit der unterhalb des Bildes oder Projektionsbildes liegenden Brust möglich.

Eine zusätzliche Vereinfachung wird erzielt, wenn in das Projektionsbild ein Verschiebevektor eingeblendet wird, der anzeigt, in welche Richtung und wie weit die Brust verschoben werden muss. Dieser Verschiebevektor kann aus dem Ort des markierten Bereiches im Röntgenbild und dem Ort des Aussparung berechnet werden.

In einer alternativen Ausgestaltung des Verfahrens wird von der Brust und einem zumindest die Aussparung umfassenden Teil der Kompressionsplatte ein Kamerabild aufgenommen und die Maske dem an einem Monitor wiedergegebenen Kamerabild derart überlagert, dass der markierte Bereich innerhalb der Aussparung angeordnet ist. Die Brust wird dann solange verschoben, bis ihre Lage und Kontur im Kamerabild zumindest annähernd mit der Lage und Kontur der dem Kamerabild überlagerten Maske übereinstimmt. Das Kamerabild wird vorzugsweise aus einer Richtung aufgenommen, die zumindest annähernd der Richtung entspricht, in der von einer Röntgenquelle oder Röntgenröhre der Mammographieeinrichtung erzeugten Röntgenstrahlen ausbreiten.

Die korrekte Positionierung wird erleichtert, wenn in das Kamerabild ein Verschiebevektor eingeblendet wird, der anzeigt, in welche Richtung und wie weit die Brust verschoben werden muss.

Die Verschiebung der virtuellen Maske in der Weise, dass der markierte Bereich innerhalb der Aussparung der Kompressionsplatte liegt, erfolgt ebenso wie die Erzeugung der virtuellen Maske und des markierten Bereiches vorzugsweise automatisch.

Hinsichtlich der Mammographieeinrichtung wird die Aufgabe gemäß der Erfindung gelöst mit einer Mammographieeinrichtung mit den Merkmalen des Patentanspruches 9. Vorteilhafte Ausgestaltungen der Mammographieeinrichtung sind in den diesem Patentanspruch untergeordneten Patentansprüchen angegeben. Die Vorteile der in Patentanspruch 9 angegebenen Mammographieeinrichtung sowie der in den Unteransprüchen angegebenen Weiterbildungen entsprechen sinngemäß den Vorteilen der diesen jeweils zugeordneten Verfahrensansprüche.

Zur weiteren Erläuterung der Erfindung wird auf die Ausführungsbeispiele der Zeichnung verwiesen. Es zeigen:
Fig. 1 und 2 alternative Ausführungsformen einer Mammographieeinrichtung gemäß der Erfindung jeweils in einer schematischen Prinzipdarstellung,
Fig. 3 ein Röntgenbild einer Brust mit einem in diesem erkennbaren Läsion,
Fig. 4 eine aus dem Röntgenbild erzeugte virtuelle Maske,
Fig. 5 ein Kamerabild der unterhalb einer mit einer Aussparung versehenen Kompressionsplatte befindlichen Brust,
Fig. 6 das Kamerabild der Brust gemäß Fig. 5 mit der diesem Kamerabild überlagerten virtuellen Maske gemäß Fig. 4,
Fig. 7 das Kamerabild der Brust und die diesem Kamerabild überlagerte virtuelle Maske nach korrekter Positionierung der Brust.

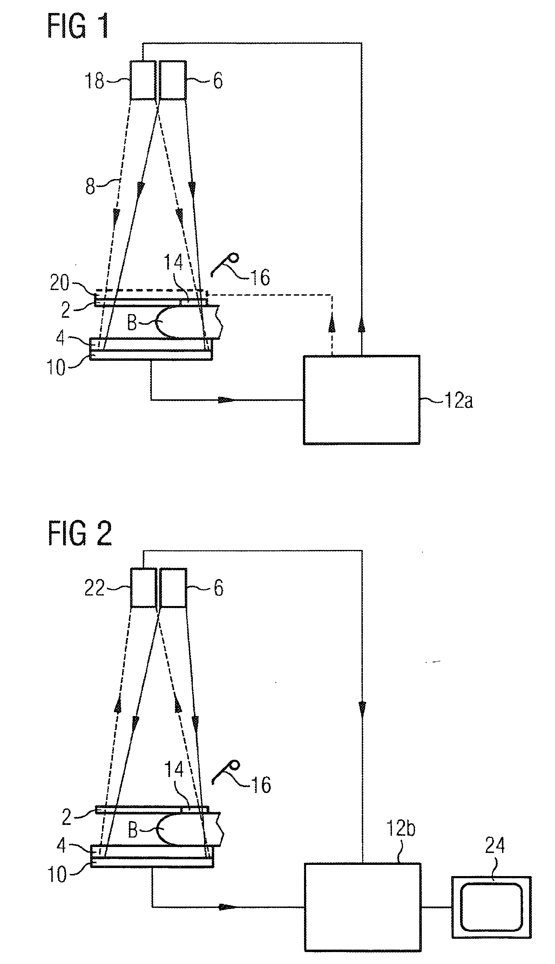

Gemäß Fig. 1 ist bei einer Mammographieeinrichtung gemäß der Erfindung eine Brust B zwischen einer Kompressionsplatte 2 und einer Lagerplatte 4 komprimiert und fixiert. Zum Erzeugen eines Röntgenbildes der Brust B werden von einer Röntgenquelle 6 Röntgenstrahlen 8 erzeugt und von einem hinter der Lagerplatte 4 angeordneten Röntgenempfänger 10 empfangen. Die vom Röntgenempfänger 10 empfangenen Bilddaten werden in einer Bildverarbeitungseinrichtung 12a verarbeitet.

Die Kompressionsplatte 2 ist mit einer rechteckförmige Aussparung 14 durch die ein Biopsieinstrument 16 in die Brust B eingeführt werden kann. Im Speicher der Bildverarbeitungseinrichtung 12a befindet sich ein Röntgenbild der Brust B, das entweder kurz vorher mit derselben Mammographieeinrichtung oder zu einem weiter zurück liegenden Zeitpunkt mit einer anderen Mammographieeinrichtung erzeugt und befündet worden ist. Aus diesem Röntgenbild wird nun in der Bildverarbeitungseinrichtung eine virtuelle Maske erzeugt, die den vom Brustgewebe abgedeckten Bildbereich im Röntgenbild wiedergibt, d. h. deren Rand mit der Kontur der Brust übereinstimmt und in der ein innerhalb des Brustgewebes liegender und zur Biopsie vorgesehener Bereich markiert ist. Dies kann automatisch durch Segmentierverfahren der digitalen Bildverarbeitung erfolgen.

Von der in der Bildverarbeitungseinrichtung 12a ermittelten virtuellen Maske wird nun mit Hilfe einer Wiedergabeeinrichtung im Bereich der Kompressionsplatte 2 ein sichtbares Bild erzeugt. Im Ausführungsbeispiel wird die Maske mit einer Projektionseinrichtung 18 für einen Betrachter sichtbar auf die Kompressionsplatte derart projiziert, d.h. visuell für den Betrachter erkennbar auf der Kompressionsplatte 2 derart abgebildet, dass der markierte Bereich innerhalb der Aussparung 14 angeordnet ist. Auf der Kompressionsplatte 2 oder der Lagerplatte 4 bzw. der Brust B ist die Maske durch Konturlinien, die die Außenkontur der Brust sowie den markierten Bereich darstellen, erkennbar.

Anhand dieses für den Betrachter sichtbaren Projektionsbildes kann nun die Brust B derart verschoben werden, dass ihre für den Betrachter sichtbare Außenkontur mit dem Rand des Projektionsbildes der Maske zumindest annähernd zusammenfällt. Dann ist die Brust korrekt zwischen Kompressionsplatte 2 und Lagerplatte 4 positioniert, da dann sichergestellt ist, dass die in der Brust B befindliche Läsion im Bereich der Aussparung 14 angeordnet ist. Somit entfällt die Notwendigkeit für die Aufnahme weiterer Röntgenbilder, die ausschließlich zu dem Zweck dienen würden, die Brust B korrekt zu positionieren.

Die visuelle Darstellung der Maske auf der Kompressionsplatte 2 oder der Lagerplatte 4 kann durch einen LCD-Projektor oder durch von einem Laser projizierte Linien erfolgen. Um die Erkennbarkeit zu erleichtern, kann die Kompressionsplatte 2 außerdem mit einem fluoreszierenden Material beschichtet sein.

Anstelle einer solchen Projektion ist es alternativ auch möglich, als Wiedergabeeinrichtung eine Leuchtquelle 20, beispielsweise ein organisches Display, auf der Kompressionsplatte 2 anzuordnen, die die Maske und den markierten Bereich wiedergibt, wie dies in der Figur gestrichelt veranschaulicht ist. Diese Leuchtquelle 20 kann, falls dies erforderlich ist, bei der nachfolgenden stereotaktischen Biopsie aus dem Strahlengang des Röntgenstrahls 8 entfernt werden.

Zur Erleichterung der korrekten Positionierung kann außerdem in das Bild oder Projektionsbild ein Verschiebevektor eingeblendet werden, der anzeigt, in welche Richtung und wie weit, d.h. welche Strecke die Brust B verschoben werden muss.

In der alternativen Ausgestaltung gemäß Fig.2 ist neben der Röntgenquelle 6 eine Kamera 22 angeordnet, die ein Kamerabild von der Brust B und einem zumindest die Aussparung 14 umfassenden Teil der Kompressionsplatte 2 aufnimmt. Das Kamerabild wird mit Hilfe einer Bildverarbeitungs- und Bildwiedergabeeinrichtung 12b auf einem Monitor 24 dargestellt. In der Bildverarbeitungs- und Bildwiedergabeeinrichtung 12b wird ebenso wie in der Bildverarbeitungseinrichtung 12a aus dem in einer vorherigen Untersuchung erzeugten Röntgenbild der Brust B eine virtuelle Maske erzeugt und dem Kamerabild derart überlagert, dass der markierte Bereich innerhalb der Aussparung 14 angeordnet ist. Die Brust B ist dann korrekt zwischen Kompressionsplatte 2 und Lagerplatte 4 positioniert, wenn ihr Kamerabild mit dem im Monitor 24 wiedergegebenen Bild der Maske annähernd zusammenfällt.

In den Fig. 1 und 2 sind sowohl die Projektionseinrichtung 18 als auch die Kamera 22 derart angeordnet, dass sich die Kompressionsplatte 2 unmittelbar im Projektionsbereich der Projektionseinrichtung 18 bzw. im Sichtfeld der Kamera 22 befinden. Alternativ hierzu kann sowohl die Projektion als auch die Kameraaufnahme über Umlenkspiegel erfolgen, so dass Projektionseinrichtung 18 bzw. Kamera 22 nicht unmittelbar neben der Röntgenquelle 6 angeordnet sein müssen.

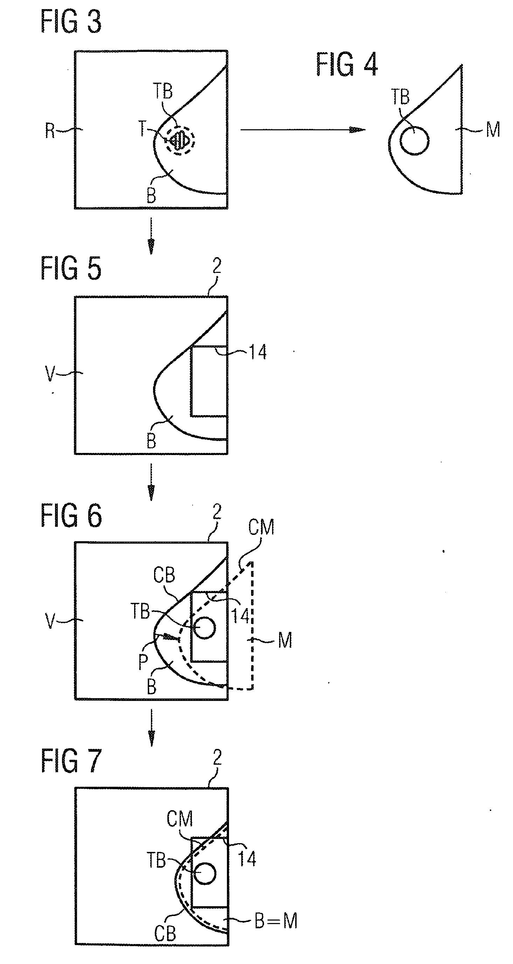

Eine Vorgehensweise gemäß der Erfindung wird nachfolgend anhand der Fig. 3 bis 7 erläutert. Gemäß Fig. 3 wird in einem ersten Schritt, in der Regel im Laufe einer Voruntersuchung, ein Röntgenbild R der Brust B unter Bedingungen erzeugt, die den Bildaufnahmebedingungen bei der stereotaktischen Biopsie entsprechen. In diesem Röntgenbild R ist eine Läsion T erkennbar. Mit Methoden der digitalen Bildverarbeitung wird durch Segmentierung eine in Fig. 4 dargestellte virtuelle Maske M erstellt, die den vom Brustgewebe abgedeckten Bildbereich wiedergibt, und in der ein innerhalb des Brustgewebes liegender Bereich TB markiert ist, innerhalb dem die Läsion T liegt.

In einem darauf folgenden Schritt wird nun die Brust B gemäß Fig. 5 in der für die Biopsie vorgesehenen Mammographieeinrichtung zwischen der Kompressionsplatte 2 und der Lagerplatte 4 positioniert und ist für den Betrachter visuell entweder unmittelbar sichtbar oder mittelbar auf einem Monitor als von einer Kamera aufgenommenes Kamerabild V mit ebenfalls erkennbarer Aussparung 14 dargestellt.

Gemäß Fig. 6 wird nun dem Kamerabild V oder unmittelbar der Kompressionsplatte 2 die Maske M derart überlagert, dass der markierte Bereich TB innerhalb der Aussparung 14 angeordnet ist. Die Brust B wird nun unterhalb der Kompressionsplatte 2 in eine in Fig. 7 dargestellte Position verschoben, in der ihre Außenkontur CB mit der Außenkontur CM der Maske M annähernd zusammenfällt. Dann ist der markierte Bereich TB und damit die innerhalb der Brust befindliche Läsion korrekt im Bereich der Aussparung 14 positioniert.

In Fig. 6 ist außerdem ein Verschiebevektor P eingeblendet, der anzeigt, in welche Richtung die Brust B unterhalb der Kompressionsplatte 2 verschoben werden muss. Als Orientierungsmarken, an die Anfangspunkt und Endpunkt des Verschiebevektors P angeheftet werden können, kann beispielsweise die Mamilla dienen.

## Patentansprüche

1. Verfahren zum Positionieren der Brust (B) für eine Biopsie in einer Mammographieeinrichtung, mit folgenden Merkmalen:
a) aus zumindest einem Röntgenbild (R) der Brust wird eine virtuelle Maske (M) erzeugt, die den vom Brustgewebe abgedeckten Bildbereich wiedergibt und in der ein innerhalb des Brustgewebes liegender und zur Biopsie vorgesehener Bereich (TB) markiert ist,
b) die Brust (B) wird für die Biopsie in der Mammographieeinrichtung zwischen einer Lagerplatte (4) und einer mit einer Aussparung (14) zum Einführen eines Biopsieinstrumentes (16) versehenen Kompressionsplatte (2) positioniert,
c) die Maske (M) und die Kompressionsplatte (2) werden derart überlagert, dass der markierte Bereich (TB) innerhalb der Aussparung (14) angeordnet ist,
d) die Brust (B) wird solange verschoben bis ihre Lage und Kontur zumindest annähernd mit der Lage und Kontur der Maske (M) übereinstimmt:

2. Verfahren nach Anspruch 1, bei dem die Erzeugung der virtuellen Maske (M) und des Bereiches (TB) automatisch erfolgt.

3. Verfahren nach Anspruch 1 oder 2, bei dem die Überlagerung von Maske (M) und Kompressionsplatte (14) automatisch erfolgt.

4. Verfahren nach einem der Ansprüche 1, 2 oder 3 bei dem ein von der Maske (M) im Bereich der Kompressionsplatte (2) sichtbares Bild erzeugt wird, und bei dem die Brust (B) solange verschoben wird, bis ihre Lage und Kontur zumindest annähernd mit der Lage und Kontur des Bildes der Maske (M) übereinstimmt.

5. Verfahren nach Anspruch 4, bei dem von der Maske (M) ein auf die Kompressionsplatte (2) projiziertes Projektionsbild erzeugt wird.

6. Verfahren nach Anspruch 4 oder 5, bei dem in das Bild bzw. in das Projektionsbild ein Verschiebevektor eingeblendet wird, der anzeigt, in welche Richtung und wie weit die Brust (B) verschoben werden muss.

7. Verfahren nach einem der Ansprüche 1, 2 oder 3 bei dem von der Brust (B) und einem zumindest die Aussparung (14) umfassenden Teil der Kompressionsplatte (2) ein Kamerabild (V) aufgenommen und die Maske (M) dem Kamerabild (V) derart überlagert wird, dass der markierte Bereich (TB) innerhalb der Aussparung (14) angeordnet ist, und bei dem die Brust (B) solange verschoben wird, bis ihre Lage und Außenkontur (CB)im Kamerabild (V) zumindest annähernd mit der Lage und Außenkontur (CM) der dem Kamerabild (V) überlagerten Maske (M) übereinstimmt.

8. Verfahren nach Anspruch 7, bei dem in das Kamerabild (V) ein Verschiebevektor (P) eingeblendet wird, der anzeigt, in welche Richtung und wie weit die Brust (B) verschoben werden muss.

9. Mammographieeinrichtung zum Durchführen des Verfahrens nach Anspruch 1, mit
a) einer Röntgenquelle (6) und einem Röntgenempfänger (10) zum Erzeugen eines Röntgenbildes (R) der Brust (B),
b) einer Bildverarbeitungseinrichtung (12a, 12b) zum Erzeugen einer virtuellen Maske (M) aus einem Röntgenbild (R), die den vom Brustgewebe abgedeckten Bildbereich wiedergibt und in der ein innerhalb des Brustgewebes liegender und zur Biopsie vorgesehener Bereich (TB) markiert ist,
c) einer Lagerplatte (4) und einer mit einer Aussparung (14) zum Einführen eines Biopsieinstrumentes (16) versehene Kompressionsplatte (2) zum Positionieren der Brust (B),
d) einer Einrichtung zum Überlagern von Maske (M) und Kompressionsplatte (2) derart, dass der markierte Bereich (TB) innerhalb der Aussparung (14) angeordnet ist.

10. Mammographieeinrichtung nach Anspruch 9, mit einer Wiedergabeeinrichtung zum Erzeugen eines im Bereich der Kompressionsplatte (2) sichtbaren Bildes von der Maske (M).

11. Mammographieeinrichtung nach Anspruch 10, bei der als Wiedergabeeinrichtung eine Projektionseinrichtung (18) zum Erzeugen eines Projektionsbildes der Maske (M) auf der Kompressionsplatte (2) vorgesehen ist.

12. Mammographieeinrichtung nach Anspruch 11, bei der die Projektionseinrichtung (18) neben der Röntgenquelle (6) angeordnet ist.

13. Mammographieeinrichtung nach einem der Ansprüche 9 bis 12, mit einer in der Bildverarbeitungseinrichtung (12a, 12b) implementierten Software zum Einblenden eines Verschiebevektors in das Projektionsbild, der anzeigt, in welche Richtung und wie weit die Brust verschoben werden muss.

14. Mammographieeinrichtung nach Anspruch 9, mit einer Kamera (22) zum Aufnehmen eines Kamerabildes von der Brust (B) und einem zumindest die Aussparung (14) umfassenden Teil der Kompressionsplatte (2) und mit einer Bildverarbeitungs- und Bildwiedergabeeinrichtung (12b) zum Wiedergeben der Maske (M) im Kamerabild derart, dass der markierte Bereich (TB) innerhalb der Aussparung (14) angeordnet ist.

15. Mammographieeinrichtung nach Anspruch 14, bei der in der Bildverarbeitungs- und Bildwiedergabeeinrichtung eine Software zum Segmentieren der Brust (B) im Kamerabild und zum Einblenden eines Verschiebevektors (V) implementiert ist, der anzeigt, in welche Richtung und wie weit die Brust (B) verschoben werden muss.

16. Mammographieeinrichtung nach einem der Ansprüche 9 bis 15, bei der die Erzeugung der virtuellen Maske (M) und des Bereiches (TB) automatisch erfolgt.

17. Mammographieeinrichtung nach einem der Ansprüche 9 bis 16, bei der die Überlagerung von Maske (M) und Kompressionsplatte (2) automatisch erfolgt.

## Claims

1. Method for positioning the breast (B) for a biopsy in a mammography facility, having the following features:
a) from at least one X-ray image (R) of the breast, a virtual mask (M) is generated which reproduces the image region covered by the breast tissue and having a region (TB) located within the breast tissue and intended for biopsy marked therein,
b) the breast (B) is positioned between a support plate (4) and a compression plate (2) in the mammography facility for the biopsy, said compression plate (2) being provided with a cutout (14) allowing insertion of a biopsy instrument (16),
c) the mask (M) and the compression plate (2) are superimposed in such a manner that the marked region (TB) is disposed within the cutout (14),
d) the breast (B) is displaced until its position and contour correspond at least approximately to the position and contour of the mask (M).

2. Method according to claim 1, in which the virtual mask (M) and the region (TB) are generated automatically.

3. Method according to claim 1 or 2, in which the mask (M) and compression plate (14) are superimposed automatically.

4. Method according to one of claims 1, 2 or 3, in which a visible image of the mask (M) is generated in the region of the compression plate (2) and in which the breast (B) is displaced until its position and contour correspond at least approximately to the position and contour of the image of the mask (M).

5. Method according to claim 4, in which a projection image of the mask (M) projected onto the compression plate (2) is generated.

6. Method according to claim 4 or 5, in which a displacement vector which indicates the direction in which and the extent to which the breast (B) has to be displaced is inserted into the image or projection image.

7. Method according to one of claims 1, 2 or 3, in which a camera image (V) is recorded of the breast (B) and a portion of the compression plate (2) containing at least the cutout (14) and the mask (M) is superimposed on the camera image (V) in such a manner that the marked region (TB) is disposed within the cutout (14) and in which the breast (B) is displaced until its position and outer contour (CB) in the camera image (V) correspond at least approximately to the position and outer contour (CM) of the mask (M) superimposed on the camera image (V).

8. Method according to claim 7, in which a displacement vector (P) which indicates the direction in which and the extent to which the breast (B) has to be displaced is inserted into the camera image (V).

9. Mammography facility for implementing the method according to claim 1, having
a) an X-ray source (6) and an X-ray receiver (10) to generate an X-ray image (R) of the breast (B),
b) an image processing facility (12a, 12b) for generating a virtual mask (M) from an X-ray image (R), said mask (M) reproducing the image region covered by the breast tissue and having a region (TB) located within the breast tissue and intended for biopsy marked therein,
c) a support plate (4) and a compression plate (2) provided with a cutout (14) allowing insertion of a biopsy instrument (16) for positioning the breast (B),
d) a facility for superimposing the mask (M) and compression plate (2) in such a manner that the marked region (TB) is disposed within the cutout (14).

10. Mammography facility according to claim 9, having a reproduction facility for generating a visible image of the mask (M) in the region of the compression plate (2).

11. Mammography facility according to claim 10, in which a reproduction facility is provided in the form of a projection facility (18) for generating a projection image of the mask (M) on the compression plate (2).

12. Mammography facility according to claim 11, in which the projection facility (18) is disposed adjacent to the X-ray source (6).

13. Mammography facility according to one of claims 9 to 12, having software implemented in the image processing facility (12a, 12b) to insert a displacement vector into the projection image, indicating the direction in which and the extent to which the breast has to be displaced.

14. Mammography facility according to claim 9, having a camera (22) for recording a camera image of the breast (B) and a portion of the compression plate (2) containing at least the cutout (14) and having an image processing and image reproduction facility (12b) for reproducing the mask (M) in the camera image in such a manner that the marked region (TB) is disposed within the cutout (14).

15. Mammography facility according to claim 14, in which software for segmenting the breast (B) in the camera image and for inserting a displacement vector (V) is implemented in the image processing and image reproduction facility, the displacement vector (V) indicating the direction in which and the extent to which the breast (B) has to be displaced.

16. Mammography facility according to one of claims 9 to 15, in which the virtual mask (M) and the region (TB) are generated automatically.

17. Mammography facility according to one of claims 9 to 16, in which the mask (M) and compression plate (2) are superimposed automatically.

## Revendications

1. Procédé pour positionner un sein (B) dans un dispositif de mammographie en vue d'une biopsie, comprenant les caractéristiques suivantes :
a) à partir d'au moins une image radiographique (R) du sein, on crée un masque virtuel (M) qui restitue la zone d'image cachée par le tissu mammaire et dans lequel est marquée une zone (TB) située à l'intérieur du tissu mammaire et destinée à la biopsie,
b) en vue de la biopsie, on positionne le sein (B) dans le dispositif de mammographie, entre une plaque d'appui (4) et une plaque de compression (2) dotée d'un creux (14) permettant d'introduire un instrument de biopsie (16),
c) on superpose le masque (M) et la plaque de compression (2) de telle manière que la zone marquée (TB) soit placée à l'intérieur du creux (14),
d) on déplace le sein jusqu'à ce que sa position et son contour coïncident, au moins approximativement, avec la position et le contour du masque (M).

2. Procédé selon la revendication 1, dans lequel la création du masque virtuel (M) et de la zone (TB) se fait automatiquement.

3. Procédé selon la revendication 1 ou 2, dans lequel la superposition du masque (M) et de la plaque de compression (14) se fait automatiquement.

4. Procédé selon l'une des revendications 1, 2 ou 3, dans lequel on produit une image visible par le masque (M) dans la région de la plaque de compression (2), et dans lequel on déplace le sein (B) jusqu'à ce que sa position et son contour coïncident, au moins approximativement, avec la position et le contour du masque (M).

5. Procédé selon la revendication 4, dans lequel le masque (M) produit une image de projection, projetée sur la plaque de compression (2).

6. Procédé selon la revendication 4 ou 5, dans lequel on insère dans l'image, ou dans l'image de projection, un vecteur de déplacement qui indique dans quelle direction et jusqu'où le sein (B) doit être déplacé.

7. Procédé selon l'une des revendications 1, 2 ou 3, dans lequel on enregistre une image de caméra (V) du sein (B) et d'une partie de la plaque de compression (2) comprenant au moins le creux (14) et on superpose le masque (M) à l'image de caméra (V) de telle manière que la zone marquée (TB) soit placée à l'intérieur du creux (14), et dans lequel on déplace le sein (B) jusqu'à ce que sa position et son contour extérieur (CB) dans l'image de caméra (V) coïncide, au moins approximativement, avec la position et le contour extérieur (CM) du masque (M) superposé à l'image de caméra (V).

8. Procédé selon la revendication 7, dans lequel on insère dans l'image de caméra (V) un vecteur de déplacement (P) qui indique dans quelle direction et jusqu'où le sein (B) doit être déplacé.

9. Dispositif de mammographie pour la mise en oeuvre du procédé selon la revendication 1, comprenant
a) une source de rayons X (6) et un récepteur de rayons X (10) pour produire une image radiographique (R) du sein (B),
b) un dispositif de traitement d'images (12a, 12b) pour créer un masque virtuel (M) à partir d'une image radiographique (R), qui restitue la zone d'image cachée par le tissu mammaire et dans lequel est marquée une zone (TB) située à l'intérieur du tissu mammaire et destinée à la biopsie,
c) une plaque d'appui (4) et une plaque de compression (2) dotée d'un creux (14) permettant d'introduire un instrument de biopsie (16), pour positionner le sein (B),
d) un dispositif pour superposer le masque (M) et la plaque de compression (2) de telle manière que la zone marquée (TB) soit placée à l'intérieur du creux (14).

10. Dispositif de mammographie selon la revendication 9, comprenant un dispositif de restitution pour produire une image visible par le masque (M) dans la région de la plaque de compression (2).

11. Dispositif de mammographie selon la revendication 10, dans lequel est prévu, comme dispositif de restitution, un dispositif de projection (18) pour produire une image de projection du masque (M) sur la plaque de compression (2).

12. Dispositif de mammographie selon la revendication 11, dans lequel le dispositif de projection (18) est disposé à côté de la source de rayons X (6).

13. Dispositif de mammographie selon l'une des revendications 9 à 12, comprenant un logiciel implémenté dans le dispositif de traitement d'images (12a, 12b) pour insérer dans l'image de projection un vecteur de déplacement qui indique dans quelle direction et jusqu'où le sein doit être déplacé.

14. Dispositif de mammographie selon la revendication 9, comprenant une caméra (22) pour enregistrer une image de caméra du sein (B) et d'une partie de la plaque de compression (2) comprenant au moins le creux (14), et comprenant un dispositif de traitement d'images et de restitution d'images (12b) pour restituer le masque (M) dans l'image de caméra de telle manière que la zone marquée (TB) soit située à l'intérieur du creux (14).

15. Dispositif de mammographie selon la revendication 14, dans lequel un logiciel est implémenté dans ledit dispositif de traitement d'images et de restitution d'images pour segmenter le sein (B) dans l'image de caméra et pour insérer un vecteur de déplacement (V) qui indique dans quelle direction et jusqu'où le sein (B) doit être déplacé.

16. Dispositif de mammographie selon l'une des revendications 9 à 15, dans lequel la création du masque virtuel (M) et de la zone (TB) se fait automatiquement.

17. Dispositif de mammographie selon l'une des revendications 9 à 16, dans lequel la superposition du masque (M) et de la plaque de compression (2) se fait automatiquement.
